# EUROPÄISCHE PATENTSCHRIFT

(11) **EP 1 743 618 B1**
(45) Veröffentlichungstag und Bekanntmachung des Hinweises auf die Patenterteilung: **19.12.2012**
(21) Anmeldenummer: 05015477.2
(22) Anmeldetag: 15.07.2005
(51) Int. Cl.: A61J 1/00, A61B 17/00, A61M 5/315, A61C 5/06

(54) **Vorrichtung zum Applizieren einer Flüssigkeit**
Device to apply a liquid
Dispositif pour appliquer un liquide

(43) Veröffentlichungstag der Anmeldung: 17.01.2007
(73) Patentinhaber: Sulzer Mixpac AG, 9469 Haag (CH)
(72) Erfinder: Sogaro, Alberto C, 61476 Kronberg (DE)
(74) Vertreter: advotec.

(56) Entgegenhaltungen:
- EP-A- 1 205 196
- WO-A1-00/23002
- DE-A1- 4 418 682
- FR-A- 1 262 088
- US-A- 4 741 737
- US-A- 6 059 570
- US-A1- 2002 160 333
- US-B1- 6 447 476
- US-B1- 6 450 810

## Beschreibung

Die Erfindung betrifft eine Vorrichtung zum Applizieren einer Flüssigkeit.

Vorrichtungen zum Applizieren einer Flüssigkeit sind in vielfältiger Weise aus der Praxis bekannt und sind beispielsweise nach Art einer Spritze ausgebildet, bei deren Betätigung ein Kolben auf einen in einem zylinderförmigen Hohlraum enthaltenen Flüssigkeitsvorrat drückt, so dass die Flüssigkeit über eine Düse ausgetragen wird siehe z.B. die WO 00/23002 und US-A-4.741.737.

Des Weiteren ist aus der Praxis eine sogenannte Minispritze bekannt, die einen im Wesentlichen zylindrischen Spritzenkörper aufweist, an dessen erster Stirnseite eine Applikationseinrichtung in Form eines Pinsels, einer Pipettierspitze oder dergleichen ausgebildet ist und an dessen zweiter Stirnseite ein hülsenartiger Vorratsbehälter angeordnet ist, der an der der Applikationseinrichtung abgewandten Seite verschlossen ist und der an einem kolbenartigen Zylinderkörper des Spritzenkörpers über einen von einer Innenwand des Vorratsbehälters vorspringenden, dichtenden Ringbund geführt ist. Der kolbenartige Zylinderkörper weist im Bereich des Vorratsbehälters eine Querbohrung auf, von der eine Längsbohrung abzweigt, die zu der Applikationseinrichtung führt. Wenn der Ringbund stromauf, d.h. an der der Applikationseinrichtung abgewandten Seite des Querkanals, den Zylinderkörper umgreift, ist ein Flüssigkeitsstrom zwischen dem Vorratsbehälter und der Applikationseinrichtung gesperrt. Wird ein Druck auf den Vorratsbehälter in Richtung der Applikationseinrichtung ausgeübt, so dass der Vorratsbehälter gegenüber dem Spritzenkörper verschoben wird und der Ringbund des Vorratsbehälters über den Querkanal gleitet. Durch den Druck des Zylinderkörpers auf die in dem Vorratsbehälter enthaltene Flüssigkeit wird die Flüssigkeit über einen Ringspalt zwischen dem Zylinderkörper und der Seitenwand des Vorratsbehälters, den Querkanal und den Längskanal zu der Applikationseinrichtung transportiert. Diese Minispritze ist aber nur zur Applikation von Einkomponentensystemen geeignet.

Der Erfindung liegt die Aufgabe zugrunde, eine Applikationsvorrichtung zu schaffen, die ein breites Anwendungsgebiet zur Applikation von Flüssigkeiten bereitstellt.

Zur Lösung dieser Aufgabe wird erfindungsgemäß eine Vorrichtung zum Applizieren einer Flüssigkeit bereitgestellt, die einen einen Aufnahmeraum begrenzenden, röhrchenartigen Applikationsbehälter umfasst, der an einem ersten Ende mit einer Applikationseinrichtung versehen ist und an seinem zweiten Ende über eine Ventileinrichtung mit einem mindestens einen Vorratsraum aufweisenden Vorratsbehälter verbunden ist. Der Vorratsbehälter ist aus einer Hülse gebildet, die an der der Applikationseinrichtung abgewandten Seite verschlossen ist und die über mindestens einen radial von einer Innenwand der Hülse vorspringenden, dichten Ringbund an einem kolbenartigen Zylinderkörper der Ventileinheit geführt ist. Der Zylinderkörper weist im Bereich der Hülse einen Querkanal auf, der mit einem Längskanal verbunden ist, der zu dem Aufnahmeraum des Applikationsbehälters führt. Der Vorratsbehälter ist gegenüber dem Applikationsbehälter derart beweglich ausgebildet, dass in einer Schließstellung des Vorratsbehälters ein Flüssigkeitsstrom zwischen dem Vorratsraum des Vorratsbehälters und dem Aufnahmeraum des Applikationsbehälters gesperrt ist und in einer Aktivierungsstellung des Vorratsbehälters der Aufnahmeraum und der Vorratsraum über den Axialkanal und den Querkanal der Ventileinheit verbunden sind.

Die Vorrichtung nach der Erfindung ist in vielfältiger Weise einsetzbar und kann beispielsweise zum Auftragen mehrkomponentiger Flüssigkeiten genutzt werden, wobei eine Komponente von dem Vorratsraum des Vorratsbehälters und die andere Komponente von dem Aufnahmeraum des Applikationsbehälters aufgenommen wird. Bei einer Aktivierung, d.h. bei einem Druck auf den Vorratsbehälter wird nach Zurücklegen einer bestimmten Wegstrecke ein Flüssigkeitsstrom zwischen dem Vorratsraum und dem Applikationsbehälter freigegeben, so dass Komponenten eines Mehrkomponentensystems in dem Applikationsbehälter vermengt werden können.

Die Komponenten eines Mehrkomponentensystems können vor der Aktivierung der Vorrichtung nach der Erfindung entweder getrennt in dem Vorratsbehälter und dem Applikationsbehälter oder auch in mittels mindestens einer Trennwand getrennten Kammern des Vorratsbehälters vorgehalten werden. In letzterem Fall wird bei Aktivierung der Vorrichtung die Trennwand verschoben, so dass alle Komponenten in den Aufnahmeraum des Applikationsbehälter verdrängt werden und sich dort vor der Applikation vermengen können.

Um ein Austreten der Komponenten aus der Vorrichtung vor deren Vermengen zu verhindern, ist es zweckmäßig, wenn im Bereich der Applikationseinrichtung eine lösbare bzw. öffenbare Verschlusseinrichtung vorgesehen ist.

Bei einer speziellen Ausführungsform der Vorrichtung nach der Erfindung ist der Applikationsbehälter aus einem elastisch verformbaren, weichen Kunststoff gefertigt. Zur Applikation kann dann auf manuelle Weise ein seitlicher Druck auf den Applikationsbehälter ausgeübt werden, so dass die darin enthaltene Flüssigkeit über die Applikationseinrichtung ausgetragen wird.

Um die Aktivierung der Vorrichtung nach der Erfindung zu erleichtern, ist im Bereich des zweiten Endes des Applikationsbehälters vorzugsweise ein Betätigungsflansch ausgebildet. Damit können beispielsweise zwei Finger einer Benutzerhand an dem Betätigungsflansch angreifen, wohingegen der Daumen der gleichen Hand zur Aktivierung auf das freie und geschlossene Stirnende des Vorratsbehälters drückt, wodurch die in dem Vorratsbehälter enthaltene Flüssigkeit über die Ventileinheit in den Applikationsbehälter gefördert wird.

Um den Vorratsbehälter vollständig entleeren zu können, hat der kolbenartige Zylinderkörper zweckmäßigerweise eine Länge, die im Wesentlichen mit der axialen Erstreckung des Vorratsbehälters korrespondiert. Damit kann der Zylinderkörper bis zu dem Boden des Vorratsbehälters in diesen eintauchen und die Flüssigkeit aus dem Vorratsbehälter verdrängen.

Vorzugsweise ist der kolbenartige Zylinderkörper von einer ringförmigen Ausnehmung der Ventileinheit begrenzt, in die der Vorratsbehälter bei einer Verschiebung in Aktivierungsstellung eintaucht.

Um eine Schließstellung des Vorratsbehälters zu definieren, kann der Ringbund des Vorratsbehälters mit einer Ringnut des Zylinderkörpers zusammenwirken, die stromauf des Querkanals angeordnet ist.

Die Applikationseinrichtung kann in vielfältiger, dem jeweiligen Anwendungsfall angepasster Form ausgebildet sein. Beispielsweise umfasst die Applikationseinrichtung einen Pinsel, eine Düse und/oder eine schwammartige Auftrageinrichtung.

Weitere Vorteile und vorteilhafte Ausgestaltungen des Gegenstandes nach der Erfindung sind der Beschreibung, der Zeichnung und den Patentansprüchen entnehmbar.

Ein Ausführungsbeispiel der Vorrichtung nach der Erfindung ist in der Zeichnung schematisch vereinfacht dargestellt und wird in der nachfolgenden Beschreibung näher erläutert. Es zeigt
- Fig. 1: einen Längsschnitt durch eine Applikationsvorrichtung für einen mehrkomponentigen Stoff in Deaktivierungsstellung; und
- Fig. 2: die Vorrichtung nach Fig. 1 in Aktivierungsstellung.

In der Zeichnung ist eine Applikationsvorrichtung 10 dargestellt, die beispielsweise im medizinischen Bereich zum Applizieren eines zweikomponentigen Systems eingesetzt werden kann. Beispielsweise kann die Vorrichtung 10 im Dentalbereich beim Einsetzen eines Keramikinlays oder dergleichen zum Auftragen eines Klebers oder dergleichen eingesetzt werden.

Die Vorrichtung 10 umfasst einen Applikationsbehälter 12, der röhrchenartig ausgebildet ist und aus einem elastisch verformbaren, weichen Kunststoff gefertigt ist. An einem ersten Ende 14 ist der Applikationsbehälter 12 mit einer Applikationseinrichtung 16 versehen, die im vorliegenden Fall mit einem pinselartigen Einsatz 18 ausgebildet ist.

An der der Applikationseinrichtung 16 abgewandten Seite ist in den Applikationsbehälter 12 eine sogenannte Ventileinheit 20 eingesetzt, die den Applikationsbehälter 12 mit einem bei der vorliegenden Darstellung umgekehrt topfförmigen Vorratsbehälter 22 verbindet. Der Vorratsbehälter 22 ist nach Art einer Hülse ausgebildet, die an ihrer Innenwand über einen dichtenden Ringbund 24 aufweist, über den sie an einem kolbenartigen Zylinderkörper 26 der Ventileinheit 20 axial verschiebbar geführt ist, und aus einem elastisch verformbaren Kunststoff gefertigt. An dem dem Applikationsbehälter 12 abgewandten Ende ist der Vorratsbehälter 22 verschlossen.

Der kolbenartige Zylinderkörper 26 umfasst in einem über einen Betätigungsflansch 28 vorspringenden Bereich einen Querkanal 30, der mit einem Axialkanal 32 verbunden ist, welcher zu einem Innenraum 34 des Applikationsbehälters 12 führt.

Des Weiteren weist die Ventileinheit 20 eine ringförmige Ausnehmung 36 auf, die den kolbenartigen Zylinderkörper 26 umschließt und in die der Vorratsbehälter 22 mit seiner Seitenwand eintaucht.

Zur Definition einer Sperrstellung, in der ein Fluidstrom zwischen einem Vorratsraum 38 des Vorratsbehälters 22 und dem Aufnahmeraum 34 des Applikationsbehälters 12 gesperrt ist, weist der Zylinderkörper 26 des Weiteren an seinem Umfang stromauf, d.h. in der Zeichnung oberhalb des Querkanals 30 eine Ringnut 40 auf, die mit dem Ringbund 24 des Vorratsbehälters 22 zusammenwirkt. In Sperrstellung rastet der Ringbund 24 in die Ringnut 40 ein.

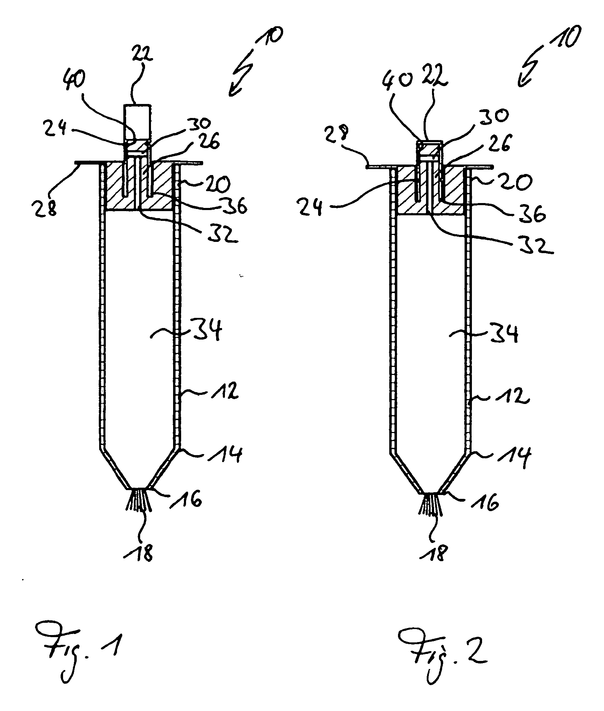

In der in Fig. 1 dargestellten Deaktivierungsstellung ist in dem Aufnahmeraum 34 des Applikationsbehälters 12 eine erste pulverförmige oder flüssige Komponente eines Mehrkomponentensystems enthalten. In dem Vorratsraum 38 des Vorratsbehälters 22 ist eine zweite, flüssige Komponente des Mehrkomponentensystems enthalten.

Durch den an dem Zylinderkörper 26 anliegenden, in die Ringnut 40 eingreifenden Ringbund 24 wird verhindert, dass die in dem Vorratsraum 38 enthaltene Flüssigkeit in den Aufnahmeraum 34 des Applikationsbehälters 12 strömt.

Bei einer Aktivierung der Vorrichtung 10 wird nach Art einer Kugelschreiberbetätigung ein axialer Druck auf den Vorratsbehälter 22 ausgeübt, wodurch der Vorratsbehälter 22 gegenüber dem Applikationsbehälter 12 in Richtung des Aufnahmeraums 34 verschoben wird und der Ringbund 24 die Öffnungen des Querkanals 30 überfährt. Durch den von dem kolbenartigen Zylinderkörper 26 ausgeübte Kraft wird die Flüssigkeit in dem Vorratsraum 38 über einen Ringspalt zwischen der Seitenwand des Vorratsbehälters 22 und dem Zylinderkörper 26, den Querkanal 30 und den Axialkanal 32 in den Aufnahmeraum 34 des Vorratsbehälters 12 verdrängt. Dort vermengt sie sich mit der anderen Komponente. Der Vorratsbehälter 22 wird so weit in Richtung des Applikationsbehälters 12 gedrückt, bis die freie Stirnseite des Zylinderkörpers 26 an dem Boden des Vorratsbehälters 22 anschlägt. Somit erfolgt eine nahezu vollständige Entleerung des Vorratsbehälters 22.

Nach einer Vermengung der beiden Komponenten in dem Aufnahmeraum 34 des Applikationsbehälters 12 kann eine hier nicht näher dargestellte Verschlusseinrichtung gelöst und das Zweikomponentensystem durch einen seitlichen manuellen Druck über die erst dann aufgesetzte Applikationseinrichtung 16 und deren Pinseleinsatz 18 an einer betreffenden Stelle appliziert werden.

Bei einer hier nicht näher dargestellten Ausführungsform weist der Vorratsbehälter zwei in axialer Richtung hintereinander angeordnete Vorratsräume auf, die durch eine verschiebbare Trennwand getrennt sind und in denen jeweils eine Komponente eines Mehrkomponentensystems angeordnet ist. Bei einer Aktivierung dieses Systems, d. h. bei einem axialen Druck auf den Vorratsbehälter wird zunächst die Komponente, die in der direkt an den Zylinderkörper grenzenden Vorratsraum enthalten ist, in den Aufnahmeraum des Applikationsbehälters verdrängt. Wenn dann die freie Stirnfläche des Zylinderkörpers an der Trennwand zwischen den beiden Vorratsräumen anschlägt, beginnt durch das Verschieben der Trennwand in axialer Richtung die Entleerung des zweiten Vorratsraums, so dass die in diesem enthaltene Komponente ebenfalls in den Aufnahmeraum des Applikationsbehälters verdrängt wird und sich mit der ersten Komponente vermengt.

## Patentansprüche

1. Vorrichtung zum Applizieren einer Flüssigkeit, umfassend einen einen Aufnahmeraum (34) begrenzenden, röhrchenartigen Applikationsbehälter (12), der an einem ersten Ende (14) mit einer Applikationseinrichtung (16) versehen ist und an einem zweiten Ende über eine Ventileinheit (20) mit einem mindestens einen Vorratsraum (38) aufweisenden Vorratsbehälter (22) verbunden ist, der aus einer Hülse gebildet ist, die an der der Applikationseinrichtung (16) abgewandten Seite verschlossen ist und die über mindestens einen radial von einer Innenwand der Hülse vorspringenden, dichtenden Ringbund (24) an einem kolbenartigen Zylinderkörper (26) der Ventileinheit (20) geführt ist, der einen im Bereich der Hülse angeordneten Querkanal (30) aufweist, der mit einem Axialkanal (32) verbunden ist, der zu dem Aufnahmeraum (34) des Applikationsbehälters (12) führt, wobei der Vorratsbehälter (22) gegenüber dem Applikationsbehälter (12) derart beweglich ausgebildet ist, dass in einer Schließstellung des Vorratsbehälters (22) ein Flüssigkeitsstrom zwischen dem Vorratsraum (38) des Vorratsbehälters (22) und dem Aufnahmeraum (34) des Applikationsbehälters (12)durch den Ringbund (24) gesperrt ist und in einer Aktivierungsstellung des Vorratsbehälters (22) der Vorratsraum (38) und der Aufnahmeraum (34) über den Querkanal (30) und den Axialkanal (32) der Ventileinheit (20) verbunden sind, wobei der Applikationsbehälter (12) aus einem elastisch verformbaren, weichen Kunststoff gefertigt ist und der kolbenartige Zylinderkörper (26) der Ventileinheit (20) von einer ringförmigen Ausnehmung (36) der Ventileinheit (20) begrenzt ist, in die der Vorratsbehälter (22) bei einer Verschiebung in Aktivierungsstellung eintaucht.

2. Vorrichtung nach Anspruch 1, **dadurch gekennzeichnet, dass** im Bereich des zweiten Endes des Applikationsbehälters (12) ein Betätigungsflansch (28) angeordnet ist.

3. Vorrichtung nach Anspruch 1 oder 2, **dadurch gekennzeichnet, dass** der kolbenartige Zylinderkörper (26) eine Länge hat, die im Wesentlichen mit der axialen Erstreckung des Vorratsbehälters (22) korrespondiert.

4. Vorrichtung nach einem der Ansprüche 1 bis 3, **dadurch gekennzeichnet, dass** der Ringbund (24) zur Definition der Schließstellung des Vorratsbehälters (22) mit einer Ringnut (40) zusammenwirkt, die stromauf des Querkanals (30) am Umfang des Zylinderkörpers (26) angeordnet ist.

5. Vorrichtung nach einem der Ansprüche 1 bis 4, **dadurch gekennzeichnet, dass** die Applikationseinrichtung (16) einen Pinsel (18) umfasst.

6. Vorrichtung nach einem der Ansprüche 1 bis 5, **dadurch gekennzeichnet, dass** die Applikationseinrichtung eine Düse umfasst.

7. Vorrichtung nach einem der Ansprüche 1 bis 5, **dadurch gekennzeichnet, dass** die Applikationseinrichtung eine schwammartige Auftrageinrichtung umfasst.

8. Vorrichtung nach einem der Ansprüche 1 bis 7, **dadurch gekennzeichnet, dass** der Aufnahmeraum (34) des Applikationsbehälters (12) eine erste Mischkomponente und der Vorratsraum (38) des Vorratsbehälters (22) eine zweite Mischkomponente eines Zweikomponentensystems aufnimmt, die bei einem Verschieben des Vorratsbehälters (22) in Aktivierungsstellung in dem Aufnahmeraum (34) des Applikationsbehälters (12) mit der ersten Mischkomponente vermengt wird.

9. Vorrichtung nach einem der Ansprüche 1 bis 8, **dadurch gekennzeichnet, dass** der Vorratsbehälter (22) mehrere Vorratsräume aufweist, die über mindestens eine verschiebbare Trennwand voneinander getrennt sind.

## Claims

1. Device for applying a liquid, comprising a tube-like application container (12) limiting a receiving space (34), the application container being equipped at a first end (14) with an application unit (16), and being connected via a valve unit (20) at a second end to a reservoir container (22) having at least one reservoir space (38), the reservoir container being formed of a sleeve that is closed at the side facing away from the application unit (16), and which is guided at a plunger-like cylindrical body (26) of the valve unit (20) via at least one sealing annular collar (24) that protrudes radially from an inner wall of the sleeve, the cylindrical body having a lateral channel (30) that is arranged in the area of the sleeve, and that is connected to an axial channel (32), which leads to the receiving space (34) of the application container (12), wherein the reservoir container (22) is formed moveable as opposed to the application container (12) such that in a closed position of the reservoir container (22) a fluid flow between the reservoir space (38) of the reservoir container (22) and the receiving space (34) of the application container (12) is blocked by the annular collar (24), and that in an activated position of the reservoir container (22) the reservoir space (38) and the receiving space (34) are connected via the lateral channel (30) and the axial channel (32) of the valve unit (20), wherein the application container (12) is made of an elastic deformable soft plastic material, and the plunger-like cylindrical body (26) of the valve unit (20) is limited by an annular recess (36) of the valve unit (20), into which the reservoir container (22) dips upon displacement in the activated position.

2. Device according to claim 1,
**characterized in that**
an actuating flange (28) is arranged in the area of the second end of the application container (12).

3. Device according to one of the claims 1 or 2,
**characterized in that**
the plunger-like cylindrical body (26) has a length essentially corresponding to the axial extension of the reservoir container (22).

4. Device according to one of the claims 1 to 3,
**characterized in that**
for the definition of the closed position of the reservoir container (22) the annular collar (24) cooperates with an annular groove (40) which is arranged upstream of the lateral channel (30) at the circumference of the cylindrical body (26).

5. Device according to one of the claims 1 to 4,
**characterized in that**
the application device (16) comprises a brush (18).

6. Device according to one of the claims 1 to 5,
**characterized in that**
the application device comprises a nozzle.

7. Device according to one of the claims 1 to 5,
**characterized in that**
the application device comprises a sponge like application device.

8. Device according to one of the claims 1 to 7,
**characterized in that**
the receiving space (34) of the application container (12) receives a first mixing component, and the reservoir space (38) of the reservoir container (22) receives a second mixing component of a two-component system, which is mixed with the first mixing component in the receiving space (34) of the application container (12) upon displacement of the reservoir container (22) in the activated position.

9. Device according to one of the claims 1 to 8,
**characterized in that**
the reservoir container (22) has several reservoir spaces, which are separated from each other by means of at least one moveable partition wall.

## Revendications

1. Dispositif pour appliquer un liquide, comprenant une cuve d'application (12) en forme d'un tube circonscrivant un espace de réception (34), cette cuve d'application ayant une unité d'application (16) à une première extrémité (14) et étant connectée à une deuxième extrémité à une unité de soupape (20) avec une cuve de réservoir (22) ayant au moins un espace de réservoir (38), cette cuve de réservoir étant formée d'une cosse qui est fermée sur le côté détourné de l'unité d'application (16) et qui est guidée sur un corps cylindrique (26) en forme d'un piston de l'unité de soupape (20) par au moins une collerette annulaire (24) d'étanchéité saillant radialement de la paroi intérieure de la cosse, ce corps cylindrique ayant dans la région de la cosse un canal transversal (30) qui est connecté à un canal axial (32) qui conduit à l'espace de réception (34) de la cuve d'application (12), dans lequel la cuve de réservoir (22) est formée mobile à l'encontre de la cuve d'application (12) de telle manière que dans une position de clôture de la cuve de réservoir (22) un écoulement de liquide entre l'espace de réservoir (38) de la cuve de réservoir (22) et l'espace de réception (34) de la cuve d'application (12) est bloqué par la collerette annulaire (24) et dans une position d'activation de la cuve de réservoir (22) l'espace de réservoir (38) et l'espace de réception (34) sont connectés par le canal transversal (30) et le canal axial (32) de l'unité soupape (20), dans lequel la cuve d'application (12) est fabriquée d'un doux matériel plastique flexible et déformable et le corps cylindrique (26) en forme d'un piston de l'unité de soupape (20) est circonscrivit par une niche annulaire (36) de l'unité de soupape (20) dans laquelle la cuve de réservoir (22) immerge en cas d'un déplacement dans la position d'activation.

2. Dispositif selon la revendication 1,
**caractérisé en ce qu'**
une bride d'actionnement (28) est disposée dans la région de la deuxième extrémité de la cuve d'application (12).

3. Dispositif selon la revendication 1 ou 2,
**caractérisé en ce que**
le corps cylindrique (26) en forme d'un piston a une longitude qui en essence correspond à la prolongation axiale de la cuve de réservoir (22).

4. Dispositif selon l'une quelconque des revendications 1 à 3,
**caractérisé en ce que**
la collerette annulaire (24) coopère avec une rainure annulaire (40) pour circonscrire la position de clôture de la cuve de réservoir (22), cette rainure annulaire étant disposée en amont du canal transversal (30) à la circonférence du corps cylindrique (26).

5. Dispositif selon l'une quelconque des revendications 1 à 4,
**caractérisé en ce que**
l'unité d'application (16) comprend une brosse (18).

6. Dispositif selon l'une quelconque des revendications 1 à 5,
**caractérisé en ce que**
l'unité d'application comprend une buse.

7. Dispositif selon l'une quelconque des revendications 1 à 5,
**caractérisé en ce que**
l'unité d'application comprend une unité d'application spongieuse.

8. Dispositif selon l'une quelconque des revendications 1 à 7,
**caractérisé en ce que**
l'espace de réception (34) de la cuve d'application (12) reçoit une première composante et l'espace de réservoir (38) de la cuve de réservoir (22) prend une deuxième composante d'un système à deux composantes, la deuxième composante étant mélangée avec la première composante dans l'espace de réception (34) de la cuve d'application (12) quand la cuve de réservoir (22) est déplacée dans la position d'activation.

9. Dispositif selon l'une quelconque des revendications 1 à 8,
**caractérisé en ce que**
la cuve de réservoir (22) comprend des plusieurs espaces de réservoir qui sont séparés par au moins une cloison mobile.
